# EUROPEAN PATENT APPLICATION

(11) **EP 2 671 571 A1**
(43) Date of publication of application: **11.12.2013**
(21) Application number: 13170372.0
(22) Date of filing: 04.06.2013
(51) Int. Cl.: A61K 9/28

(54) **Controlled-release formulations of clarithromycin**

(30) Priority: 05.06.2012 TR 201206529
(71) Applicant: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(57) **Abstract**

This invention relates to a controlled-release formulation, composed of a core and a coating layer, and comprising clarithromycin or a pharmaceutically acceptable salt, solvate, or polymorph of clarithromycin.

## Description

### Field of Invention

The present invention relates to a novel pharmaceutical formulation of clarithromycin or a pharmaceutically acceptable salt, solvate or hydrate of clarithromycin. The present invention more particularly relates to stable formulations of clarithromycin which provide a desired level of release.

### Background of Invention

Clarithromycin is an orally-administered antibiotic, belonging to the macrolide group. Similar to other antibiotics of the macrolide group, clarithromycin inhibits RNA-mediated synthesis of bacterial protein by binding to the 50S subunit of 70S ribosome.

The chemical name of clarithromycin belonging to the macrolide group is (3R,4S,5S,6R,7R,9R,11 R,12R,13S,14R)-6-[(2S,3R,4S,6R)-4-(dimethylamino)-3-hydroksi-6-methyloxan-2-yl]oxy-14-ethyl-12,13-dihydroxy-4-[(2R,4R,5S,6S)-5-hydroxy-4-methoxy-4,6-dimethyloxan-2-yl]oxy-7-methoxy-3,5,7,9,11,13-hexamethyl-oxacyclotetradecane-2,10-dione with the chemical structure shown below with Formula 1.

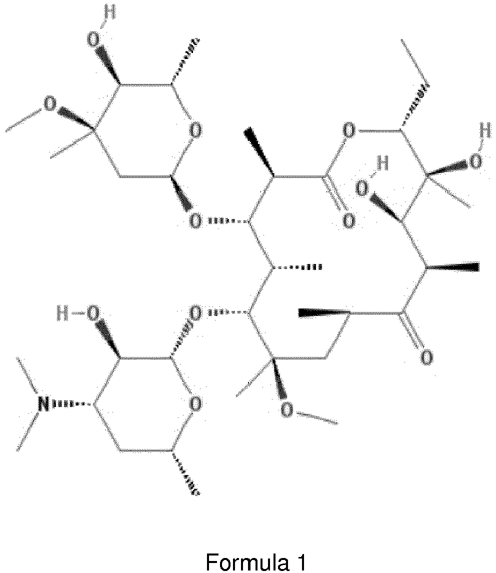

A controlled-release formulation of clarithromycin is marketed under the name Klacid MR^{®} and is orally administered once a day. It contains 500 mg clarithromycin as the active agent.

The clarithromycin molecule is disclosed in the application EP0041355.

The patent EP1302205, in turn, discloses a controlled release formulation comprising a clarithromycin citrate salt, hydrophilic polymer, binder, filler and a lubricant, wherein said formulation is obtained by dispersing said macrolide in an organic solvent, adding an aqueous solution comprising an equimolar amount of citric acid to the macrolide, and evaporating the organic solvent.

The patent WO9846239 discloses a pharmaceutical composition of clarithromycin for extended release in the gastrointestinal environment. Said composition comprises (a) clarithromycin, and (b) a pharmaceutically acceptable polymer in an amount of about 5% to about 50% by weight of the composition. When administered orally, the bioavailability of clarithromycin becomes substantially equivalent to that of immediate release compositions of clarithromycin, and the clarithromycin composition induces statistically significantly lower mean fluctuation index in the plasma than an immediate release composition thereof.

It is a desired feature to obtain an active pharmaceutical agent in a controlled-release form in the treatment of a number of diseases. The term controlled release may be defined as reaching desired plasma levels of an active agent of interest throughout a determined period of time, and providing the drug release at a uniform and constant rate. Thus, the drug administration frequency can be lowered.

There are various difficulties associated with developing a controlled-release formulation. One of the problems encountered is dose dumping, as a result of which a drug obtained is released too rapidly. Obtaining intended solubility profiles is also difficult, i.e. it is difficult to control the release rate. For this reason, there may occur fluctuations in the plasma concentrations of active agents, which may lead to toxicity. In addition, daily alterations may be possible for the active agent in plasma.

Various formulations are available, which have been developed with clarithromycin. There are various problems, however, associated with clarithromycin formulations.

A problem associated with controlled-release systems is the difficulty in achieving proper release profiles. Particularly the dose dumping may lead to undesired outcomes in terms of patients. Dose dumping is one of the most important drawbacks of extended-release dosage forms. The most significant criterion of dose dumping under *in vitro* conditions is the amount of active agent released at an earlier time point. Any release of the active agent in a higher amount than desired at a determined time interval may lead to uncontrollable damages in patients.

The production of controlled-release tablets of clarithromycin and similar active agents with low solubility rates and the provision of desired release profiles in these tablets are quite difficult. Generating release amounts in desired ranges bears vital importance in terms of patients. Obtaining a desired release profile depends on the convenience of excipients to be selected.

Clarithromycin is susceptible to external influences under normal conditions. Ambient humidity and temperature influence the stability of a product. Thus, stability-related problems are encountered when these values exceed a certain level. Failing to prepare a formulation with convenient excipients leads to stability problems.

Another problem encountered in the development of controlled-release formulations is the adhesion of tablets to production machinery, equipment and punches. There are also difficulties encountered in the flowability of granules which are obtained.

Accordingly, there is a need for controlled-release formulations of clarithromycin or a pharmaceutically-acceptable salt thereof, which would overcome the problems referred to hereinabove, and would provide drug plasma concentrations equivalent to or better than those of the currently-used immediate-release tablet formulations. The formulation according to this invention provides a novel controlled-release formulation, not disclosed in the relevant prior art yet.

The controlled-release formulation according to this invention both provides plasma levels comparable to the immediate-release form thereof, and gives an advantageous feature in terms of administering the active drug less frequently, e.g. once or twice a day.

Although coatings are applied to clarithromycin formulations which are yet finished for stability purposes, they sometimes become inadequate for protecting the product against humidity. The coating selected and its feature are quite significant.

Carrying out the film coating process as required in an accurate manner is very crucial in terms of ensuring the stability of the clarithromycin molecule and preventing the adhesion of tablets to equipment. A preferred coating must both provide protection against ambient effects to ensure stability, and not cause any problems encountered in film coatings, such as wrinkling surfaces, blister and bubble formation, efflorescence, peeling, etc. At this point, the proper selection and compatibility of the coating excipient and the plasticizer are crucial.

Due to the reasons specified above, there is a need for a stable pharmaceutical formulation of clarithromycin or pharmaceutically acceptable salts, solvates, or hydrates thereof, providing a proper content uniformity and a desired release profile.

### Object and Brief Description of Invention

The present invention provides a controlled-release formulation of clarithromycin, eliminating all aforesaid problems and bringing additional advantages to the relevant prior art.

Accordingly, the main object of the present invention is to obtain controlled-release formulations comprising clarithromycin, which are stable and provide a desired release profile.

Another object of the present invention is to obtain a controlled-release formulation which would not stick to equipment and punches during production.

A further object of the present invention is to develop a controlled-release formulation which would not lead to flowability problems during production.

Another object of the present invention is to prevent any dose dumping from occurring in the pharmaceutical formulation by means of a coating applied.

A further object of the present invention is to obtain a controlled-release formulation having a desired dissolution rate by means of proper coating excipients employed.

A controlled-release pharmaceutical formulation, which is composed of a core and a coating layer, and comprises clarithromycin or a pharmaceutically acceptable salt, solvate, or polymorph thereof has been developed to carry out all objects, referred to above and to emerge from the following detailed description.

In a preferred embodiment of the present invention, said novelty is embodied in that
a. the total core weight comprises
   i. 1 to 10% by weight of talk,
   ii. 1 to 5% by weight of hydroxypropyl methylcellulose of 4000 CPS viscosity, and
b. the total coating weight comprises
   i. 1 to 60% by weight of polyvinyl alcohol,
   ii. 1 to 30% by weight of polyethylene glycol.

In a preferred embodiment of the present invention, said excipient(s) comprise(s) at least one or a mixture of controlled release-providing agents, fillers, binders, lubricants.

In a preferred embodiment of the present invention, said controlled release-providing agent contains at least one or a mixture of polymethacrylate, glyceryl behenate, polyvinylpyrrolidone (povidone), cross-linked polyvinylpyrrolidone, hydroxypropyl methylcellulose (HPMC), hydroxypropyl cellulose (HPC), carboxymethyl cellulose (CMC), methyl cellulose (MC), ethyl cellulose (EC) and other cellulose derivatives, polyethylene oxide and gelatin.

In a preferred embodiment of the present invention, said filler is at least one or a properly-proportioned mixture of microcrystalline cellulose and lactose.

In another preferred embodiment according to the present invention, said binders comprise at least one or a mixture of polymethacrylate, glyceryl behenate, polyvinylpyrrolidone (povidone), cross-linked polyvinylpyrrolidone, hydroxypropyl methylcellulose (HPMC), hydroxypropyl cellulose (HPC), carboxymethyl cellulose (CMC), methyl cellulose (MC), ethyl cellulose and similar cellulose derivatives, polyethylene oxide, gelatin.

In another preferred embodiment of the present invention, the lubricant is magnesium stearate. The proportion of magnesium stearate to the total core weight is 0.1 to 5%. Thus, this component contributes to the flowability and to the prevention of sticking to the punches, machinery and equipment.

A preferred embodiment of the present invention provides a method for preparing said formulation, this method comprising the steps of
a. mixing together clarithromycin, filler and the controlled-release agent,
b. forming wet granulation using an alcohol-water mixture,
c. wet milling and drying,
d. sieving the granules,
e. adding talk and magnesium stearate into the sieved granular powder mixture and mixing the same,
f. compressing into tablets, and
g. coating the tablets.

In a preferred embodiment of the present invention, said formulation comprises the following ingredients:
a. core, comprising a total of
   i. 1 to 60% by weight of clarithromycin
   ii. 1 to 5% by weight of hydroxypropyl methylcellulose of 4000 CPS viscosity
   iii. 1 to 60% by weight of lactose
   iv. 1 to 10% by weight of talk
   v. 0.1 to 5% by weight of magnesium stearate
b. coating layer, comprising a total of
   i. 1 to 60% by weight of polyvinyl alcohol
   ii. 1 to 30% by weight of polyethylene glycol.

This invention is used particularly for preventing or treating such diseases in humans which are caused by staphylococcus aureus, streptococcus pneumoniae, streptococcus pyogenes, haemophilus influenzae, haemophilus parainfluenzae, moraxella catarrhalis, mycoplasma pneumoniae, chlamydia pneumoniae, mycobacterium avium complex (MAC) bacteria, mycobacterium avium, mycobacterium intracellulare, helicobacter pylori, streptococcus agalactiae, streptococcus strains (group C, F, G), viridans streptococcus group, bordetella pertussis, legionella pneumophila, pasteurella multocida, clostridium perfringens, peptococcus niger, propionibacterium acnes and prevotella (Bacteriodes melaninogenica).

In another preferred embodiment of the present invention, said pharmaceutical formulation is in the form of a tablet.

### Detailed Description of Invention

### Example

| **Ingredients** | **Amount (%) (w/w)** |
|---|---|
| **core** | |
| clarithromycin | 1 - 60 |
| hydroxypropyl methylcellulose | 1 - 5 |
| lactose | 1 - 60 |
| talk | 1 - 10 |
| magnesium stearate | 0.1 - 5 |
| **coasting** | 0.1 - 3 |

Clarithromycin, lactose and hydroxypropyl methylcellulose are mixed together. Wet granulation is formed using an alcohol-water mixture. Following wet milling and drying, formed granules are sieved. To the sieved granules are added talk and magnesium stearate and the resulting mixture is mixed. At the final step, the mixture is compressed into tablets and coated.

With the present invention developed, a stable controlled-release formulation is surprisingly obtained, by which clarithromycin is uniformly dispersed therein, a desired release profile is achieved, and the aforesaid problems are solved. The film coating process is realized as desired in an accurate manner and the stability of clarithromycin molecule is ensured. The amount of the controlled-release agent is lowered by using hydroxypropyl methylcellulose having 4000 CPS nominal viscosity so that the sticking phenomenon is prevented from occurring to the machinery and equipment and a formulation is obtained in which the flowability is kept substantially high during production. With the formulation developed, any dose dumping risk is eliminated.

The coating which comprises a plasticizer both ensures stability, and inhibits ambient influences, as well as prevents the problems which are encountered in film coatings, such as wrinkling surfaces, blister and bubble formation, efflorescence, peeling, etc. At this point, the proper selection and compatibility of the coating excipient and the plasticizer are crucial.

In result, flowability, easy release from equipment, ideal release profile and stability are provided by means of proper excipients used in the core and coating of the controlled-release formulation.

It is also possible to use the following additional excipients in the formulation.

A binder, e.g. at least one or a mixture of polyvinylpyrrolidone, gelatin, sugars, glucose, natural gums, gums, synthetic celluloses, polymethacrylate, hydroxypropyl methylcellulose, hydroxypropyl cellulose, carboxymethyl cellulose, methyl cellulose, and other cellulose derivatives, etc..

A glidant, e.g. at least one or a mixture of colloidal silicone dioxide, aluminum silicate, etc..

A lubricant, e.g. at least one or a mixture of sodium stearyl fumarate, polyethylene glycol, stearic acid, metal stearates, boric acid, sodium chloride benzoate and acetate, sodium or magnesium lauryl sulfate, etc..

A colorant, e.g. at least one or a mixture of food, drug, and cosmetic (FD & C) dyes (FD & C blue, FD & C green, FD & C red, FD & C yellow, FD & C lake), ponceau, indigo drug & cosmetic (D & C) blue, indigotine FD & C blue, carmoisine indigotine (indigo Carmine); iron oxides (e.g. iron oxide red, yellow, black), quinoline yellow, flame red, brilliant red (carmine), carmoisine, sunset yellow, etc..

The protection scope of the present invention is set forth in the annexed claims and cannot be restricted to the illustrative disclosures given above, under the detailed description. Any alternative embodiments to be produced by those skilled in the art according to the basic principles, which are under the protection scope as set forth in the claims, shall be an infringement of the present invention.

## Claims

1. A controlled-release formulation, composed of a core and a coating layer, and comprising clarithromycin or a pharmaceutically acceptable salt, solvate, or polymorph of clarithromycin, **characterized in that**
a. the total core weight comprises
i. 1 to 10% by weight of talk,
ii. 1 to 5% by weight of hydroxypropyl methylcellulose of 4000 CPS viscosity, and
b. the total coating weight comprises
i. 1 to 60% by weight of polyvinyl alcohol,
ii. 1 to 30% by weight of polyethylene glycol.

2. A controlled-release tablet formulation according to Claim 1, further comprising at least one or a mixture of controlled release-providing agents, fillers, binders, lubricants as an excipient.

3. A controlled-release tablet formulation according to any of the preceding claims, wherein said controlled release-providing agent contains at least one or a mixture of polymethacrylate, glyceryl behenate, polyvinylpyrrolidone (povidone), cross-linked polyvinylpyrrolidone, hydroxypropyl methylcellulose (HPMC), hydroxypropyl cellulose (HPC), carboxymethyl cellulose (CMC), methyl cellulose (MC), ethyl cellulose (EC) and other cellulose derivatives, polyethylene oxide, gelatin.

4. A pharmaceutical formulation according to any of the preceding claims, wherein said filler is at least one or a properly-proportioned mixture of microcrystalline cellulose and lactose.

5. A pharmaceutical formulation according to any of the preceding claims, wherein said binders comprise at least one or a mixture of polymethacrylate, glyceryl behenate, polyvinylpyrrolidone (povidone), cross-linked polyvinylpyrrolidone, hydroxypropyl methylcellulose (HPMC), hydroxypropyl cellulose (HPC), carboxymethyl cellulose (CMC), methyl cellulose (MC), ethyl cellulose and similar cellulose derivatives, polyethylene oxide, and gelatin.

6. A pharmaceutical formulation according to any of the preceding claims, comprising magnesium stearate as the lubricant.

7. A pharmaceutical formulation according to any of the preceding claims, wherein the proportion of magnesium stearate to the total core weight is 0.1 to 5%.

8. A method for preparing a pharmaceutical formulation according to any of the preceding claims, comprising the steps of
a. mixing together clarithromycin, filler and a controlled-release agent,
b. forming wet granulation using an alcohol-water mixture,
c. wet milling and drying,
d. sieving the granules,
e. adding talk and magnesium stearate into the sieved granular powder mixture and mixing the same,
f. compressing into tablets, and
g. coating the tablets.

9. A pharmaceutical formulation according to any of the preceding claims, consisting of the following ingredients:
a. core, comprising a total of
i. 1 to 60% by weight of clarithromycin,
ii. 1 to 5% by weight of hydroxypropyl methylcellulose of 4000 CPS viscosity,
iii. 1 to 60% by weight of lactose,
iv. 1 to 10% by weight of talk,
v. 0.1 to 5% by weight of magnesium stearate,
b. coating layer, comprising a total of
i. 1 to 60% by weight of polyvinyl alcohol,
ii. 1 to 30% by weight of polyethylene glycol.

10. A pharmaceutical formulation according to any of the preceding claims for use in mammalians and particularly in humans for preventing or treating such diseases which are caused by staphylococcus aureus, streptococcus pneumoniae, streptococcus pyogenes, haemophilus influenzae, haemophilus parainfluenzae, moraxella catarrhalis, mycoplasma pneumoniae, chlamydia pneumoniae, mycobacterium avium complex (MAC) bacteria, mycobacterium avium, mycobacterium intracellulare, helicobacter pylori, streptococcus agalactiae, streptococcus strains (group C, F, G), viridans streptococcus group, bordetella pertussis, legionella pneumophila, pasteurella multocida, clostridium perfringens, peptococcus niger, propionibacterium acnes and prevotella (Bacteriodes melaninogenica).

11. A pharmaceutical formulation according to any of the preceding claims, this formulation being in the form of a tablet.
